# EUROPEAN PATENT SPECIFICATION

(11) **EP 3 643 348 B1**
(45) Date of publication and mention of the grant of the patent: **08.03.2023**
(21) Application number: 18202749.0
(22) Date of filing: 26.10.2018
(51) Int. Cl.: A61M 16/06

(54) **NASAL PILLOW MASK WITH COMBINED POKA-YOKE COUPLING SYSTEM AND MAGNETIC CONNECTING SYSTEM**
NASENKISSENMASKE MIT KOMBINIERTEM POKA-YOKE-KOPPLUNGSSYSTEM UND MAGNETISCHEM VERBINDUNGSSYSTEM
MASQUE À COUSSINET NASAL AVEC UN SYSTÈME DE COUPLAGE ANTI-ERREUR COMBINÉ À UN SYSTÈME DE CONNEXION MAGNÉTIQUE

(43) Date of publication of application: 29.04.2020
(73) Proprietor: Air Liquide Medical Systems, 92160 Antony (FR)
(72) Inventor: MASSERDOTTI, Fulvio, 25073 Bovezzo (IT); ALBERICI, Luca, 25073 Bovezzo (IT); MASSARO, Paolo, 25073 Bovezzo (IT)
(74) Representative: Air Liquide

(56) References cited:
- EP-A1- 2 679 266
- EP-A1- 2 679 267
- WO-A1-2010/041966
- WO-A1-2014/015382
- WO-A1-2018/007966
- US-A1- 2009 044 808
- US-A1- 2015 335 846

## Description

The present invention concerns a respiratory mask, namely a nasal pillow mask, comprising a mask body and a nasal cushion detachably attached to the mask body by means of a poka-yoke coupling system combined with a magnetic connecting system, and a respiratory gas delivering assembly comprising a gas source, preferably a CPAP or BPAP device, and such a mask.

Respiratory masks are commonly used for treating sleep apnea or other respiratory troubles or diseases. A respiratory mask comprises a mask body, also called a "frame" or "shell", a flexible cushion fixed to the mask body and that comes into contact with the face of the patient, and a headgear, also called a "harness", comprising straps for maintaining the mask in position on the face of a patient, while the mask is used. Different kinds of masks exist, such as nasal masks covering only the nose, facial mask or full-face masks covering the nose and the mouth, nasal pillow masks comprising prongs inserted into the nostrils... Examples for respiratory masks, especially nasal pillow masks, are given by WO-A-2014/015382, US-A-2015/0335846, WO-A-2017/042717, WO-A-2014/077708, EP-A-2679267, US-A-6431172, US-A-2009/032026, US-A-2011/209709 and US-A-2010/108073.

Mask elements, such as the flexible cushion carrying the two prongs and the mask body, have to be regularly uncoupled for cleaning and/or replacement purposes.

However, correctly positioning and securing the flexible cushion to the mask body of a nasal pillow mask can be a problem for many users, i.e. patients. For instance, elderly people often encounter difficulties in correctly positioning the flexible cushion with respect to the mask body, so that it is not rare that the flexible cushion is coupled upside down.

The problem is that a bad coupling of the cushion to the mask body results in impaired tightness and gas leaks, which negatively impact the efficacy of the respiratory treatment provided to the patient.

A goal of the present invention is to propose an improved nasal pillow mask allowing an easy fixing and tight securing of the flexible cushion to the mask body, in a detachable manner, which further prohibits the cushion from being coupled upside down, thereby avoiding leaks and impaired tightness.

The solution of the present invention concerns a nasal pillow mask comprising a mask body comprising a central opening and an outer surface, and a flexible nasal cushion comprising a pair of hollow nasal prong, said nasal cushion being detachably coupled to the mask body, that the nasal pillow mask further comprises:
a) a poka-yoke coupling system comprising at least :
   - a finger element arranged on the nasal cushion, said finger element comprising a free end with a hook structure, and
   - a lodging traversing the mask body and comprising an exit orifice, wherein the hook structure of the finger element cooperates with the outer surface of the mask body, when the finger element is inserted into the lodging, for retaining the nasal cushion integrally coupled to the mask body, i.e. integrally fixed to, and
b) a magnetic connecting system for detachably securing the flexible nasal cushion to the mask body.

The poka-yoke coupling system according to the present invention ensures a double function that comprises an efficient coupling of the cushion to the mask body and always in the right way, thanks to its mistake-proof structure (i.e. poka-yoke), whereas the magnetic connecting system according to the present invention reinforces the efficacy of the coupling of the cushion to the mask body.

In other words, the poka-yoke coupling system cooperates with the magnetic connecting system thereby obtaining a combined effect, namely an easy fixing and tight securing of the flexible cushion to the mask body, and a prohibition of the cushion from being wrongly assembled, while avoiding or minimizing leaks and impaired tightness.

A mask according to the present invention combining a poka-yoke coupling system and a magnetic connecting system can further comprise one or more of the following additional features.
- the hook structure of the finger element cooperates with a gripping area of the mask body, said gripping area being located on the outer surface of the mask body, immediately around the exit orifice of the lodging.
- the hook structure traverses the mask body and abuts against the gripping area located immediately around the exit orifice of the lodging traversing the mask body.
- the hook structure comprises an abutment surface and a ramp structure.
- the abutment surface of the hook structure abuts against the gripping area located immediately around the central opening of the mask body.
- the hook structure is carried by a rigid ring structure integrally secured to the nasal cushion.
- the rigid ring structure and the finger structure are made of a polymer material, such as polycarbonate (PC), polypropylene (PP), polyamide (PA), acrylonitrile butadiene styrene (ABS) or polystyrene (PS) or polybutylene (PBT).
- the finger element of the nasal cushion and the lodging traversing the mask body form a male/female coupling structure.
- the inner surface of the mask body comprises a protruding wall at least partially surrounding the central orifice.
- the nasal cushion comprises a peripheral edge and at least a peripheral groove arranged in at least a part of said peripheral edge.
- the nasal cushion comprises a peripheral edge surrounding a cushion large opening.
- the nasal cushion further comprises a first inner compartment in fluid communication with the cushion large opening.
- the protruding wall is lodged into said peripheral groove, when the nasal cushion is integrally coupled to the mask body, so that said protruding wall cooperates with said peripheral groove for ensuring a gas-tight connection in-between.
- the protruding wall and the peripheral groove have complementary shapes.
- the central opening of the mask body has a circular shape.
- the mask body comprises a second inner compartment.
- the central opening of the mask body is in fluid communication with the second inner compartment of the mask body.
- the nasal cushion and said mask body define an inner gas chamber, i.e. inner gas compartment, said inner gas chamber being in fluid communication with the inner passage of each prong, and further with the central opening of the mask body.
- the inner gas chamber of the mask comprises the first inner compartment of the nasal cushion and the second inner compartment of the mask body.
- the magnetic connecting system comprises at least a first magnet arranged on the flexible nasal cushion and at least a second magnet arranged on the mask body.
- the magnetic connecting system comprises a first magnet arranged on the flexible nasal cushion and a second magnet arranged on the mask body.
- a first magnet arranged on the flexible nasal cushion faces a second magnet arranged on the mask body, when the flexible nasal cushion is secured to the mask body.
- according to another embodiment, the magnetic connecting system comprises at least two first magnets arranged on the flexible nasal cushion and at least two second magnets arranged on the mask body.
- the magnets have a square or circular section, or any other suitable shape.
- the first magnet arranged on the flexible nasal cushion and the second magnet arranged on the mask body are in a given magnetic orientation, i.e. N/S or S/N, for ensuring a magnetic attraction.
- the first magnet is arranged into a first lodging of the flexible nasal cushion.
- the second magnet is arranged into a second lodging of the mask body, preferably a cap or similar is closing the second lodging.
- the first magnet is retained in the first lodging of the flexible nasal cushion by the rigid ring structure, such as a rigid annular element, that carries the hook structure.
- the first magnet and the hook structure are symmetrically-arranged with respect to the cushion large opening of the flexible nasal cushion, i.e. arranged on diametrically-opposite sides of the cushion large opening of the flexible nasal cushion.
- the mask body comprises two elongated arms symmetrically arranged with respect to the mask body.
- the elongated arms project away from the mask body.
- the mask body and the two elongated arms are molded in one-piece.
- the mask body further comprises a headgear coupled to the two elongated arms. The headgear is used for fixing and maintaining the mask on the face of the user, i.e. the patient.
- the elongated arms have preferably a ribbon-like structure, i.e. a flat elongated structure.
- each elongated arms has a length of between 5 cm and 30 cm, typically of less than about 20 cm.
- each elongated arms has a width of between 0,5 cm and 5 cm, typically of less than about 4 cm.
- each elongated arm comprises at least two opposite surfaces comprising an inner surface and an outer surface, the inner surface being arranged toward the face of the patient, when the patient carries the mask, preferably at least a part of the inner surface comes into contact with the face of the patient, in use, typically with the cheek regions of the patient.
- the headgear comprises flexible straps.
- the headgear further comprises flexible straps forming a loop-structure, i.e. a ring-like structure.
- the flexible straps are made of a woven material, i.e. woven fibers or fabric material, preferably a polymer fabric, such as UBL Lycra Polyurethane.
- the flexible sleeve structures are (slightly) stretchable.
- the mask body and the elongated arms are made one-piece of a polymer material, preferably as polycarbonate (PC), polypropylene (PP), polyamide (PA), acrylonitrile butadiene styrene (ABS) or polystyrene (PS) or polybutylene (PBT).
- the flexible cushion is detachable, i.e. can be removed for being replaced or cleaned, when worn out or dirty.
- the flexible cushion is made of silicone or the like.
- the flexible cushion comprises two hollow prongs forming hollow conducts for delivering a respiratory gas, such as air or oxygen-enriched air, to the nostrils of the patient.
- the flexible cushion comprises two hollow prongs that are sized and configured for entering/being inserted into the nostrils of the patient, when said patient wears the mask.
- each prong of the flexible cushion comprises an inner passage with an inlet orifice and an outlet orifice.
- the inner compartment of the nasal cushion is further in fluid communication with the inner passage of each prong, i.e. via the inlet orifice of each prong, so that gas can pass, i.e. circulate, from said inner compartment to the inner passages of the two prongs.
- each prong comprises an enlarged head, preferably an enlarged head having a (almost) tronconical shape
- each prong comprises an enlarged head comprising the outlet orifice.
- the mask body is rigid.
- the mask body is rigid and made of plastic material, such as polycarbonate, polypropylene, acrylonitrile butadiene styrene, polyamide, polystyrene or the like.
- the mask comprises a plan of symmetry, i.e. it has a symmetrical structure or shape.
- the two elongated arms project laterally on each side of the mask body, i.e. left and right sides (i.e. with respect to the plan of symmetry of the mask).
- each elongated arm comprises a proximal end fixed to the mask body and a free distal end.
- the inner passages of the two hollow prongs are in fluid communication with the inner gas chamber of the mask body.
- the hollow prongs are flexible.
- the mask body further comprises a hollow elbow connector fluidly connected to the central opening, e.g., plugged into the central opening.
- the hollow elbow connector is in fluid communication with the inner gas chamber, through the central opening, for delivering the respiratory gas to said inner gas chamber, i.e. for allowing gas travelling into the lumen of the elbow connector entering into the inner gas chamber.
- the hollow elbow connector further comprises one or several venting ports for venting to the atmosphere CO₂-enriched gases expired by the patient.
- said one or several venting ports, such as a large opening or venting port, used for venting to the atmosphere CO₂-enriched gases expired by the patient is covered (i.e. closed) by a gas-permeable membrane element, such as a woven or fabric mesh material (i.e. woven fibers) or the like.
- the hollow elbow connector is fluidly connected to the downstream end of a flexible hose (i.e. tube or conduct) so that the gas circulating into said flexible hose can be delivered to the lumen of the hollow elbow connector.
- the flexible hose is made of plastic material.
- the flexible hose further comprises an upstream end in fluid communication with a hollow swivel connector.

Furthermore, the invention also concerns a respiratory gas delivering assembly comprising:
- a gas source, preferably a CPAP or BPAP device, for delivering a pressurized respiratory gas, such as air under pressure (i.e. pressure > 1 cm H₂O), and
- a nasal pillow mask according to the present invention, e.g., a nasal pillow mask comprising two hollow prongs entering/inserted into the nostrils of the patient, when said patient wears the mask.

Further, a flexible hose, such as a plastic tube or the like, is used for conveying the gas delivered by the gas source to the respiratory mask thereby delivering said gas to the airways of the patient. Preferably, the flexible hose is fluidly connected to a hollow connector, preferably an elbow connector, fixed to the mask body.

Other features, aspects and advantages of the present invention will become apparent from the following detailed description when taken in conjunction with the accompanying drawings which illustrates, by way of examples, the present invention, among which:
- Figure 1 shows an embodiment of a nasal pillow mask according to the present invention,
- Figures 2 and 3 show front and rear views of the mask body of the mask of Figure 1,
- Figures 4 and 5 are partial views of the flexible cushion of the mask of Figure 1, and
- Figure 6 shows the poka-yoke coupling system and the magnetic connecting system of the mask of Figure 1.

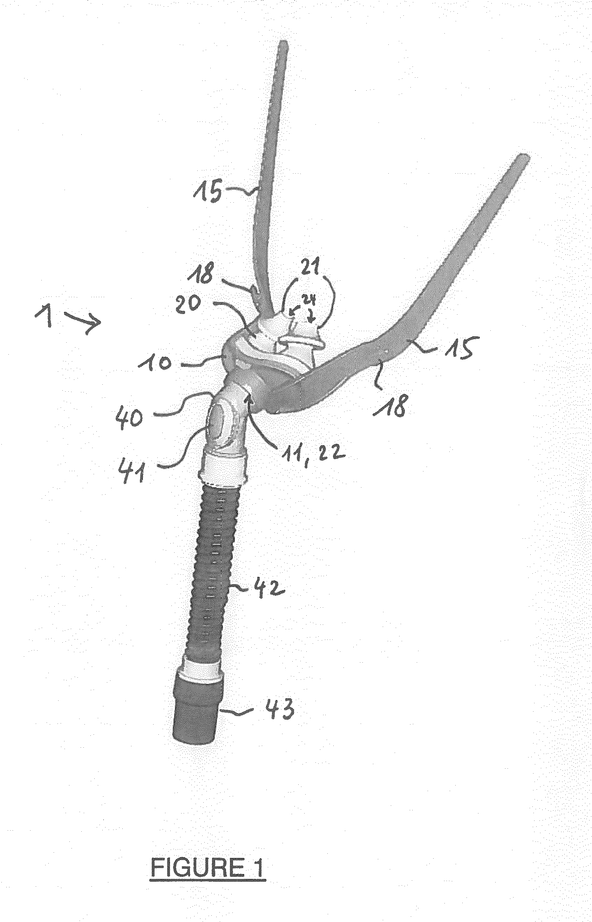

Figure 1 shows an embodiment of a respiratory mask 1 according to the present invention, namely a nasal pillow mask, that comprises a flexible cushion 20 detachably fixed to a rigid mask body 10, also called a mask "frame" or "shell".

The nasal pillow mask 1 has a generally symmetrical shape, i.e. comprises a (vertically-oriented) plan of symmetry.

As shown in Figures 1-3, the mask body 10 comprises two elongated arms 15 laterally-projecting on each side of the mask body 10, i.e. left and right sides (with respect to the plan of symmetry of the mask). The two lateral arms 15 have an elongated flat structure, such as a ribbon-like structure, and each comprise two opposite surfaces, namely an outer surface and an inner surface. The inner surface is in contact with the face of the patient, when he/she wears the mask 1, in particular with his/her cheek regions, whereas the outer surface is outwardly-oriented. The two lateral arms 15 and the mask body 10 are integral, preferably they are molded in one-piece, for instance made of a plastic material, such as polycarbonate (PC), polypropylene (PP), polyamide (PA), acrylonitrile butadiene styrene (ABS) or polystyrene (PS) or polybutylene (PBT).

The flexible cushion 20 is made for instance of silicone, whereas the mask body 10 and the two lateral arms 15 are made of plastic material, such as polycarbonate, polypropylene, acrylonitrile butadiene styrene, polyamide or polystyrene.

Further, the flexible cushion 20 comprising two flexible hollow prongs 21 for delivering the respiratory gas (e.g., air, O₂-enriched air...) to the nostrils of the patient.

Each flexible hollow prong 21 comprises an inner passage 22 with an inlet orifice 23 for conveying the gas towards the patient, and further an outlet orifice 24 arranged on the free enlarged head of each prong 21 for delivering the gas to a nostril.

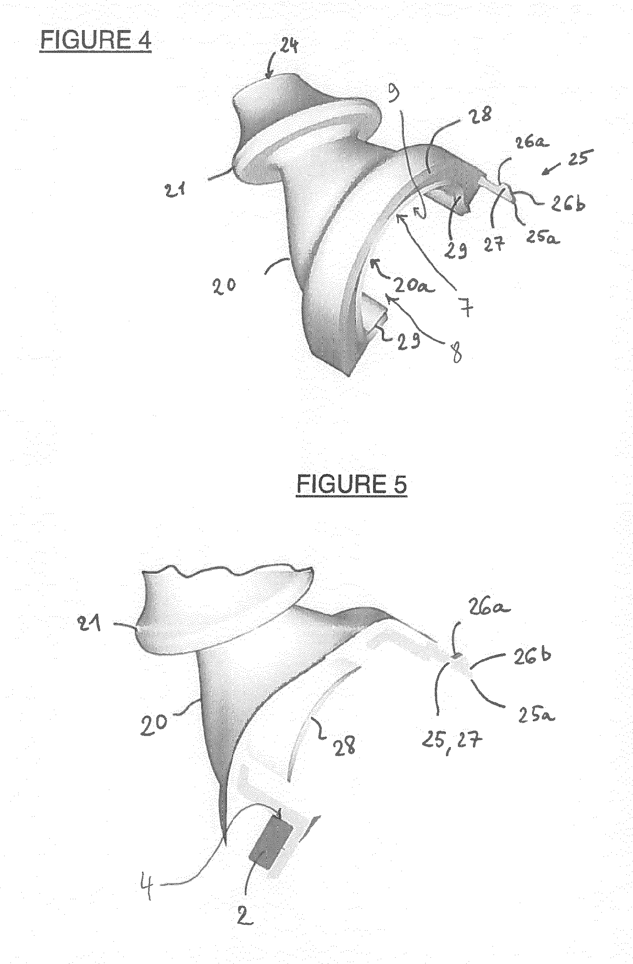

The free enlarged head of each prong 21 has a generally-tronconical shape as shown in Fig. 4 and 5. In use, the free enlarged heads of the prongs 21 are inserted into the nostrils of the patient and deliver gas therein.

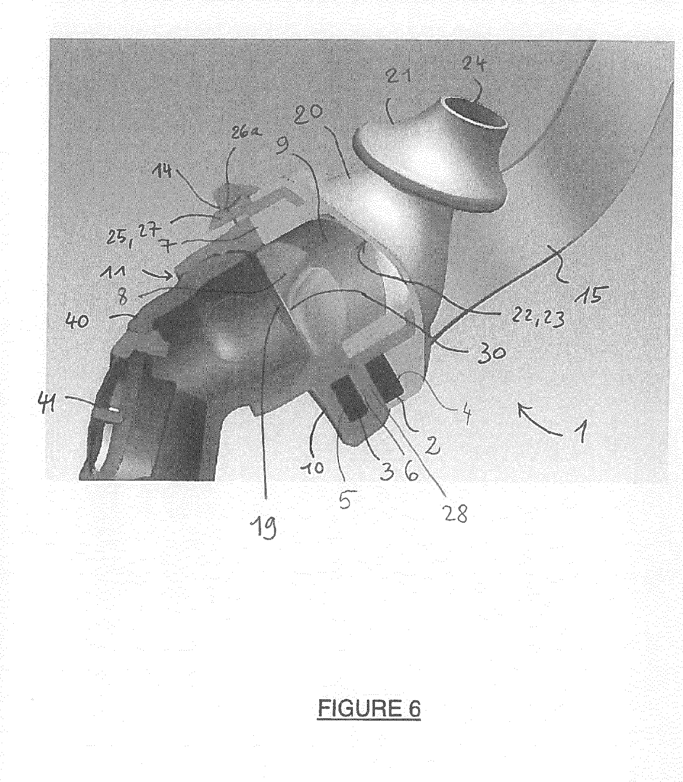

Further, the nasal cushion 20 comprises a peripheral edge 7 surrounding a cushion large opening 8 and a first inner compartment 9 in fluid communication with the cushion large opening 8, as shown in Figure 6.

Similarly, the mask body 10 comprises a second inner compartment 19, the central opening 11 of the mask body 10 being in fluid communication with said second inner compartment 19, as visible on Figure 6.

The first inner compartment 9 of the nasal cushion 20 defines with the second inner compartment 19 of the mask body 10 an inner gas chamber 30 containing respiratory gas.

In other words, the nasal cushion 20 define with the rear part, i.e. the central opening 11, of the mask body 10, an inner gas chamber 30 or gas compartment that is in fluid communication, on the one hand, with the inner passage 22 of each prong 21, and, on the other hand, with the central opening 11 of the mask body 10 for recovering the respiratory gas entering into the mask via the central opening 11 of the mask body 10 and distributing it to the prongs 21, during the inspiration phases of the patient. In contrast, at least a part of the CO₂-enriched gas expired by the patient during its expiration phases, travels in the other way, before being vented to the atmosphere through the venting ports 41 of an elbow connector 40 that is plugged into the central opening 11 of the mask body 10.

Indeed, as shown in Fig. 1-3, the mask body 10 comprises a central opening 11 forming a gas inlet 11 that receives a hollow tubular connector 40 that conveys into its lumen and delivers a respiratory gas, such as air, into the inner gas chamber 30.

The hollow connector 40, preferably an elbow connector, comprising one or several venting ports 41, is fixed to the gas inlet 22 of the mask body 10 for providing gas to the inner gas chamber. The venting ports 41 are used for venting to the atmosphere, the CO₂-enriched gases expired by the patient. The hollow elbow connector 40 is itself fluidly connected to the downstream end of a flexible hose 42 (i.e. tube) so that the respiratory gas conveyed by said flexible hose 42 can be provided to the lumen of the connector 40. The flexible hose 42 further comprises an upstream end in fluid communication with a hollow swivel connector 43 that can be connected to a source of respiratory gas, such as CPAP or BPAP device, delivering a pressurized respiratory gas, such as air under pressure (i.e. pressure > 1 cm H₂O).

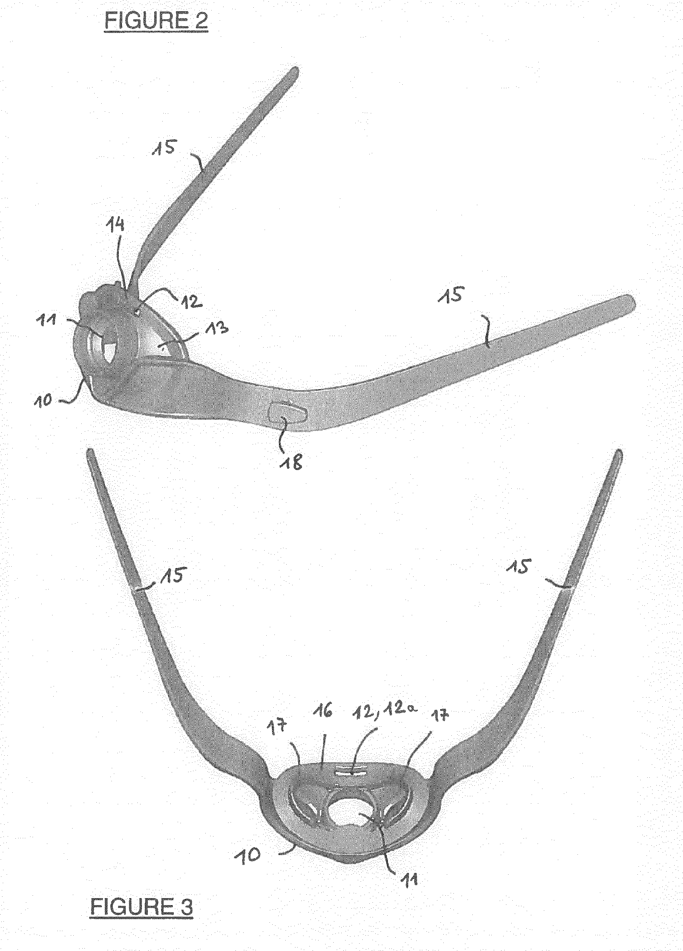

A headgear (not shown) made of flexible straps or similar, preferably made of fabric, preferably of a polymer woven material or the like, can be detachably secured to the two elongated arms 15 that project away from the mask body 10 by means of any suitable headgear fixing system. For example, the headgear fixing system can comprise pin elements 18 arranged on the elongated arms 15, as shown in Fig. 1 & 2, that cooperate with traversing holes arranged in flexible sleeve structures attached to the straps of the headgear so that the elongated arms 15 are inserted into the flexible sleeve structures, whereas the pin elements 18 are inserted into the traversing holes for maintaining the elongated arms 15 in position and integral with the flexible sleeve structures of the headgear. Other headgear fixing systems can be used, such as hooks cooperating with loops, or the like.

According to the present invention, the mask 1 further comprises a combination of a poka-yoke coupling system 12, 25 and of a magnetic connecting system 2, 3 cooperating together for ensuring efficient and integral attachment of the cushion 20 to the mask body 10 without any risk of bad coupling, i.e. upside down, of the cushion 20 to the mask body 10.

The poka-yoke coupling system 12, 25 comprises, as shown in Fig. 2-6, on the one hand, a finger element 25, i.e. elongated structure, arranged on the nasal cushion 20, said finger element 25 comprising a free end 25a with a hook structure 27 and, on the other hand, a lodging 12, i.e. a passage or the like, traversing the mask body 10 and comprising an exit orifice 12a, that cooperate together as below explained.

More precisely, the hook structure 27 of the finger element 25 cooperates with the outer surface 13 of the mask body 10, preferably with a gripping area 14 of the body 10, when the finger element 25 is inserted into the lodging 12, for retaining the nasal cushion 20 integrally coupled to the mask body 10.

The gripping area 14 is located on the outer surface 13 of the mask body 10, immediately around the exit orifice 12a of the lodging 12, i.e. in a surrounding region or zone arranged at a distance of less than 5 mm around the exit orifice 12a.

The hook structure 27 comprises an abutment surface 26a and a ramp structure 26b. The abutment surface 26a cooperates with the gripping area 14 of the mask body 10, i.e. the abutment surface 26a abuts against the gripping area 14, for ensuring that the cushion 20 is secured to the mask body 10, whereas the ramp structure 26b helps the insertion of the finger element 25 through the lodging 12.

Advantageously, the hook structure 27 is carried by a rigid ring structure 28, also called an annular rim, integrally secured to the nasal cushion 20. For instance, the silicone-made cushion 20 can be over-molded around the rigid ring structure 28, or they can also be snap-fitted, glued or thermo-welded together.

The rigid ring 28 provides an increased rigidity to the flexible cushion 20 that eases its coupling to the mask body 10, but also anchors the finger element 25.

Preferably, the rigid ring structure 28 and the finger structure 25 are made of a polymer material, such as polycarbonate (PC), polypropylene (PP), polyamide (PA), acrylonitrile butadiene styrene (ABS) or polystyrene (PS) or polybutylene (PBT).

Furthermore, for ensuring efficient gas tightness, the inner surface 16 of the mask body 10 carries, on its rear surface as shown in Fig. 3, a protruding wall 17, i.e. a little wall or tiny portions of walls, that partially or totally surrounds the central orifice 11, whereas the nasal cushion 20 comprises a peripheral edge 20a comprising a peripheral groove 29, that are configured to have complementary shapes, i.e. matching shapes.

When the nasal cushion 20 is integrally coupled to the mask body 10, the protruding wall 17 of the mask body 10 is lodged into the peripheral groove 29 of the cushion 20 thereby forming a seal, i.e. a gas-tight connection in-between.

Furthermore, the magnetic connecting system 2, 3 of a mask 1 according to the present invention comprises a pair of magnets, namely a first magnet 2 arranged on the flexible nasal cushion 20 and a second magnet 3 arranged on the mask body 10.

The first magnet 2 faces the second magnet 3, when the flexible nasal cushion is secured to the mask body, thereby ensuring a magnetic connection, i.e. attraction, between them. In other words, the first magnet 2 arranged on the flexible nasal cushion 20 and the second magnet 3 arranged on the mask body 10 are arranged in a given magnetic orientation, i.e. N/S or S/N, for ensuring a magnetic attraction between them.

Of course, depending on the embodiment, more than two magnets can be used, for instance two pairs of magnets.

The magnets 2, 3 can have a square or circular section, or any other suitable shape.

Preferably, the first magnet 2 is arranged into a first lodging 4 of the flexible nasal cushion 20, whereas the second magnet 3 is arranged into a second lodging 5 of the mask body 10.

Advantageously, a cap 6 or similar is closing the second lodging 5, thereby maintaining the second magnet 3 into the second lodging 5, whereas the first magnet 2 is retained in the first lodging 4 of the flexible nasal cushion 20 by the rigid ring structure 28, such as a rigid annular element, that carries the hook structure 25, 27.

Preferably, as shown in Figure 6, the first magnet 2 and the hook structure 25, 7 are symmetrically-arranged with respect to the cushion large opening 8 of the flexible nasal cushion 20, i.e. arranged on diametrically-opposite sides of the cushion large opening 8 of the flexible nasal cushion 20.

The respiratory mask 1 according to the present invention is useable for treating respiratory failures or disorders, such as sleep apnea or the like.

## Claims

1. Nasal pillow mask (1) comprising a mask body (10) comprising a central opening (11) and an outer surface (13), a flexible nasal cushion (20) comprising a pair of hollow nasal prong (21), said nasal cushion (20) being detachably coupled to the mask body (10), and a poka-yoke coupling system (12, 25) comprising at least :
- a finger element (25) arranged on the nasal cushion (20), said finger element (25) comprising a free end (25a) with a hook structure (27), and
- a lodging (12) traversing the mask body (10) and comprising an exit orifice (12a),
wherein the hook structure (27) of the finger element (25) cooperates with the outer surface (13) of the mask body (10), when the finger element (25) is inserted into the lodging (12), for retaining the nasal cushion (20) integrally coupled to the mask body (10),
**characterized in that** the nasal pillow mask further comprises a magnetic connecting system (2, 3) for detachably securing the flexible nasal cushion (20) to the mask body (10).

2. Nasal pillow mask according to Claim 1, **characterized in that** the hook structure (27) of the finger element (25) cooperates with a gripping area (14) of the mask body (10), said gripping area (14) being located on the outer surface (13) of the mask body (10), immediately around the exit orifice (12a) of the lodging (12).

3. Nasal pillow mask according to Claim 2, **characterized in that** the hook structure (27) traverses the mask body (10) and abuts against the gripping area (14) located immediately around the exit orifice (12a) of the lodging (12) traversing the mask body (10).

4. Nasal pillow mask according to any one of the preceding Claims, **characterized in that** the hook structure (27) comprises an abutment surface (26a) and a ramp structure (26b).

5. Nasal pillow mask according to Claim 4, when dependent on claim 2, **characterized in that** the abutment surface (26a) of the hook structure (27) abuts against the gripping area (14) located immediately around the central opening (11) of the mask body (10).

6. Nasal pillow mask according to any one of the preceding Claims, **characterized in that** the hook structure (27) is carried by a rigid ring structure (28) integrally secured to the nasal cushion (20).

7. Nasal pillow mask according to any one of the preceding Claims, **characterized in that** the magnetic connecting system (2, 3) comprises at least a first magnet (2) arranged on the flexible nasal cushion (20) and at least a second magnet (3) arranged on the mask body (10).

8. Nasal pillow mask according to any one of the preceding Claims, **characterized in that** a first magnet (2) arranged on the flexible nasal cushion (20) faces a second magnet (3) arranged on the mask body (10), when the flexible nasal cushion (20) is secured to the mask body (10).

9. Nasal pillow mask according to any one of Claims 7 or 8, **characterized in that** the first magnet (2) is arranged into a first lodging (4) of the flexible nasal cushion (20), and the second magnet (3) is arranged into a second lodging (5) of the mask body (10), preferably a cap (6) is closing the second lodging (5).

10. Nasal pillow mask according to Claim 9, when dependent on claim 6, **characterized in that** the first magnet (2) is retained in the first lodging (4) of the flexible nasal cushion (20) by the rigid ring structure (28).

11. Nasal pillow mask according to Claim 1, **characterized in that** the mask body (10) comprises two elongated arms (15) symmetrically arranged with respect to the mask body (10), preferably molded in one-piece.

12. Nasal pillow mask according to Claim 1, **characterized in that** the mask body (10) is made of a polymer material, such as Polycarbonate (PC), Polypropylene (PP), Polyamide (PA) and Polybutylene (PBT).

13. Nasal pillow mask according to Claim 1, **characterized in that** the nasal cushion (20) is made of silicone material.

14. Nasal pillow mask according to Claim 11, **characterized in that** it further comprises a headgear coupled to the two elongated arms (15).

15. Respiratory gas delivering assembly comprising a gas source, preferably a CPAP or BPAP device, for delivering a pressurized respiratory gas, such as air under pressure, and a nasal pillow mask (1) according to any one of the preceding claims.

## Patentansprüche

1. Nasenkissenmaske (1), umfassend einen Maskenkörper (10), der eine zentrale Öffnung (11) und eine Außenseite (13) umfasst, ein flexibles Nasenkissen (20), das ein Paar hohle Nasenprongs (21) umfasst, wobei das Nasenkissen (20) lösbar mit dem Maskenkörper (10) gekoppelt ist, und ein Poka-Yoke-Kopplungssystem (12, 25), das mindestens umfasst:
- ein Fingerelement (25), das an dem Nasenkissen (20) angeordnet ist, wobei das Fingerelement (25) ein freies Ende (25a) mit einer Hakenstruktur (27) umfasst und
- eine Aufnahme (12), die durch den Maskenkörper (10) durchgeht und eine Austrittsöffnung (12a) umfasst,
wobei die Hakenstruktur (27) des Fingerelements (25) mit der Außenseite (13) des Maskenkörpers (10) zusammenwirkt, wenn das Fingerelement (25) in die Aufnahme (12) eingesetzt wird, um das Nasenkissen (20) einstückig mit dem Maskenkörper (10) gekoppelt zu halten,
**dadurch gekennzeichnet, dass** die Nasenkissenmaske ferner ein magnetisches Verbindungssystem (2,3) zum lösbaren Befestigen des flexiblen Nasenkissens (20) an dem Maskenkörper (10) umfasst.

2. Nasenkissenmaske nach Anspruch 1, **dadurch gekennzeichnet, dass** die Hakenstruktur (27) des Fingerelements (25) mit einer Greiffläche (14) des Maskenkörpers (10) zusammenwirkt, wobei sich die Greiffläche (14) an der Außenseite (13) des Maskenkörpers (10) befindet, unmittelbar rund um die Austrittsöffnung (12a) der Aufnahme (12).

3. Nasenkissenmaske nach Anspruch 2, **dadurch gekennzeichnet, dass** die Hakenstruktur (27) den Maskenkörper (10) durchquert und an die Greiffläche (14) anstößt, die sich unmittelbar rund um die Austrittsöffnung (12a) der Aufnahme (12) befindet, die durch den Maskenkörper (10) durchgeht.

4. Nasenkissenmaske nach einem der vorhergehenden Ansprüche, **dadurch gekennzeichnet, dass** die Hakenstruktur (27) eine Anschlagfläche (26a) und eine Rampenstruktur (26b) umfasst.

5. Nasenkissenmaske nach Anspruch 4, in Abhängigkeit von Anspruch 2, **dadurch gekennzeichnet, dass** die Anschlagfläche (26a) der Hakenstruktur (27) an die Greiffläche (14) anschlägt, die sich unmittelbar rund um die zentrale Öffnung (11) des Maskenkörpers (10) befindet.

6. Nasenkissenmaske nach einem der vorhergehenden Ansprüche, **dadurch gekennzeichnet, dass** die Hakenstruktur (27) von einer starren Ringstruktur (28) getragen wird, die einstückig an dem Nasenkissen (20) befestigt ist.

7. Nasenkissenmaske nach einem der vorhergehenden Ansprüche, **dadurch gekennzeichnet, dass** das magnetische Verbindungssystem (2, 3) mindestens einen ersten Magneten (2) umfasst, der an dem flexiblen Nasenkissen (20) angeordnet ist, und mindestens einen zweiten Magneten (3), der an dem Maskenkörper (10) angeordnet ist.

8. Nasenkissenmaske nach einem der vorhergehenden Ansprüche, **dadurch gekennzeichnet, dass** ein erster Magnet (2), der an dem flexiblen Nasenkissen (20) angeordnet ist, einem zweiten Magneten (3), der an dem Maskenkörper (10) angeordnet ist, zugewandt ist, wenn das flexible Nasenkissen (20) an dem Maskenkörper (10) befestigt ist.

9. Nasenkissenmaske nach einem der Ansprüche 7 oder 8, **dadurch gekennzeichnet, dass** der erste Magnet (2) in einer ersten Aufnahme (4) des flexiblen Nasenkissens (20) angeordnet ist, und der zweite Magnet (3) in einer zweiten Aufnahme (5) des Maskenkörpers (10) angeordnet ist, vorzugsweise ein Verschluss (6) die zweite Aufnahme (5) verschließt.

10. Nasenkissenmaske nach Anspruch 9, in Abhängigkeit von Anspruch 6, **dadurch gekennzeichnet, dass** der erste Magnet (2) durch die starre Ringstruktur (28) in der ersten Aufnahme (4) des flexiblen Nasenkissens (20) gehalten wird.

11. Nasenkissenmaske nach Anspruch 1, **dadurch gekennzeichnet, dass** der Maskenkörper (10) zwei längliche Arme (15) umfasst, die mit Bezug auf den Maskenkörper (10) symmetrisch angeordnet sind, vorzugsweise einstückig.

12. Nasenkissenmaske nach Anspruch 1, **dadurch gekennzeichnet, dass** der Maskenkörper (10) aus einem Polymermaterial, wie Polycarbonat (PC), Polypropylen (PP), Polyamid (PA) und Polybutylen (PBT) hergestellt ist.

13. Nasenkissenmaske nach Anspruch 1, **dadurch gekennzeichnet, dass** das Nasenkissen (20) aus Silikonmaterial besteht.

14. Nasenkissenmaske nach Anspruch 11, **dadurch gekennzeichnet, dass** sie ferner eine Maskenhalterung umfasst, die mit den zwei länglichen Armen (15) gekoppelt ist.

15. Atemgaszufuhranordnung, umfassend eine Gasquelle, vorzugsweise ein CPAP- oder BPAP-Gerät, zum Zuführen eines druckbeaufschlagten Atemgases, wie Druckluft, und eine Nasenkissenmaske (1) nach einem der vorhergehenden Ansprüche.

## Revendications

1. Masque à coussinet nasal (1) comprenant un corps de masque (10) comprenant une ouverture centrale (11) et une surface extérieure (13), un coussinet nasal (20) flexible comprenant une paire de saillies nasales (21) creuses, ledit coussinet nasal (20) étant accouplé de manière détachable au corps de masque (10), et un système d'accouplement anti-erreur (12, 25) comprenant au moins :
- un élément formant doigt (25) placé sur le coussinet nasal (20), ledit élément formant doigt (25) comprenant une extrémité libre (25a) avec une structure de crochet (27), et
- un logement (12) traversant le corps de masque (10) et comprenant un orifice de sortie (12a),
la structure de crochet (27) de l'élément formant doigt (25) coopérant avec la surface extérieure (13) du corps de masque (10) lorsque l'élément formant doigt (25) est inséré dans le logement (12), pour retenir le coussinet nasal (20) en accouplement solidaire avec le corps de masque (10),
**caractérisé en ce que** le masque à coussinet nasal comprend, en outre, un système de raccordement magnétique (2, 3) pour assujettir de manière détachable le coussinet nasal (20) flexible au corps de masque (10).

2. Masque à coussinet nasal selon la revendication 1, **caractérisé en ce que** la structure de crochet (27) de l'élément formant doigt (25) coopère avec une région d'accrochage (14) du corps de masque (10), ladite région d'accrochage (14) étant située sur la surface extérieure (13) du corps de masque (10), immédiatement autour de l'orifice de sortie (12a) du logement (12).

3. Masque à coussinet nasal selon la revendication 2, **caractérisé en ce que** la structure de crochet (27) traverse le corps de masque (10) et vient en appui contre la région d'accrochage (14) située immédiatement autour de l'orifice de sortie (12a) du logement (12) traversant le corps de masque (10).

4. Masque à coussinet nasal selon l'une quelconque des revendications précédentes, **caractérisé en ce que** la structure de crochet (27) comprend une surface d'appui (26a) et une structure de plan incliné (26b).

5. Masque à coussinet nasal selon la revendication 4, lorsqu'elle dépend de la revendication 2, **caractérisé en ce que** la surface d'appui (26a) de la structure de crochet (27) vient en appui contre la région d'accrochage (14) située immédiatement autour de l'ouverture centrale (11) du corps de masque (10).

6. Masque à coussinet nasal selon l'une quelconque des revendications précédentes, **caractérisé en ce que** la structure de crochet (27) est supportée par une structure annulaire rigide (28) assujettie solidairement au coussinet nasal (20).

7. Masque à coussinet nasal selon l'une quelconque des revendications précédentes, **caractérisé en ce que** le système de raccordement magnétique (2, 3) comprend au moins un premier aimant (2) placé sur le coussinet nasal (20) flexible et au moins un second aimant (3) placé sur le corps de masque (10).

8. Masque à coussinet nasal selon l'une quelconque des revendications précédentes, **caractérisé en ce qu'**un premier aimant (2) placé sur le coussinet nasal (20) flexible fait face à un second aimant (3) placé sur le corps de masque (10) lorsque le coussinet nasal (20) flexible est assujetti au corps de masque (10).

9. Masque à coussinet nasal selon l'une quelconque des revendications 7 et 8, **caractérisé en ce que** le premier aimant (2) est placé dans un premier logement (4) du coussinet nasal flexible (20), et le second aimant (3) est placé dans un second logement (5) du corps de masque (10), un chapeau (6) fermant de préférence le second logement (5).

10. Masque à coussinet nasal selon la revendication 9, lorsqu'elle dépend de la revendication 6, **caractérisé en ce que** le premier aimant (2) est retenu dans le premier logement (4) du coussinet nasal (20) flexible par la structure annulaire rigide (28).

11. Masque à coussinet nasal selon la revendication 1, **caractérisé en ce que** le corps de masque (10) comprend deux bras allongés (15) placés symétriquement par rapport au corps de masque (10), de préférence moulés en une seule pièce.

12. Masque à coussinet nasal selon la revendication 1, **caractérisé en ce que** le corps de masque (10) est fait d'un matériau polymère, tel que le polycarbonate (PC), le polypropylène (PP), le polyamide (PA) et le polybutylène (PBT).

13. Masque à coussinet nasal selon la revendication 1, **caractérisé en ce que** le coussinet nasal (20) est fait d'un matériau de silicone.

14. Masque à coussinet nasal selon la revendication 11, **caractérisé en ce qu'**il comprend, en outre, une coiffure accouplée aux deux bras allongés (15).

15. Ensemble d'administration de gaz respiratoire comprenant une source de gaz, de préférence un dispositif de VPPC ou VPPDN, pour administrer un gaz respiratoire sous pression, tel que de l'air sous pression, et un masque à coussinet nasal (1) selon l'une quelconque des revendications précédentes.
